# EUROPEAN PATENT SPECIFICATION

(11) **EP 3 749 193 B1**
(45) Date of publication and mention of the grant of the patent: **27.04.2022**
(21) Application number: 19707942.9
(22) Date of filing: 07.02.2019
(51) Int. Cl.: A61B 5/339, A61B 5/287, A61B 5/343, A61B 5/06, A61B 5/053, A61B 34/20

(54) **SYSTEM AND METHOD FOR MAPPING AND CHARACTERIZING THE CORONARY VASCULATURE FOR EPICARDIAL ABLATIONS**
SYSTEM UND VERFAHREN ZUR ABBILDUNG UND CHARAKTERISIERUNG DER KORONAREN VASKULATUR FÜR EPIKARDIALE ABLATIONEN
SYSTÈME ET PROCÉDÉ DE CARTOGRAPHIE ET DE CARACTÉRISATION DU SYSTÈME VASCULAIRE CORONAIRE POUR ABLATIONS ÉPICARDIQUES

(30) Priority: 07.02.2018 US 201862627727 P
(43) Date of publication of application: 16.12.2020
(73) Proprietor: Boston Scientific Scimed Inc., Maple Grove, Minnesota 55311 (US)
(72) Inventor: SULKIN, Matthew S., New Brighton, Minnesota 55112 (US); SHUROS, Allan C., St. Paul, Minnesota 55116 (US)
(74) Representative: Pfenning, Meinig & Partner mbB
(86) International application number: PCT/US2019/017110
(87) International publication number: WO 2019/157219

(56) References cited:
- US-A1- 2011 201 915
- US-A1- 2014 024 911
- US-A1- 2015 223 726
- US-A1- 2017 303 816
- US-A1- 2017 372 474
- US-B2- 8 326 419

## Description

### CROSS-REFERENCE TO RELATED APPLICATION

This application claims priority to Provisional Application No. 62/627,727, filed February 7, 2018.

### TECHNICAL FIELD

The present disclosure relates to medical devices and methods for cardiac mapping. More specifically, the disclosure relates to systems and methods for generating epicardial vascular cardiac maps.

### BACKGROUND

Use of minimally invasive procedures, such as catheter ablation, to treat a variety of heart conditions, such as supraventricular and ventricular arrhythmias, is becoming increasingly more prevalent. Such procedures involve the mapping of electrical activity in the heart (e.g., based on cardiac signals), such as at various locations on the endocardium and/or epicardium surface ("cardiac mapping"), to identify the site of origin of the arrhythmia followed by a targeted ablation of the site. To perform such cardiac mapping a catheter with one or more electrodes can be inserted into the patient's body.

In embodiments, for example, endocardial ablation of ventricular tachycardia can be difficult when the critical isthmus is located in the mid-myocardium or sub-epicardium, and ablation is generally accomplished, in these situations, using an epicardial approach to transect isthmus and terminate arrhythmia. It is generally desirable to avoid ablating on certain epicardium structures such as, for example, fat, nerves, veins, arteries, and/or the like. For example, ablating on coronary arteries should be avoided to prevent pericardial effusion and to minimize further ischemic heart disease. The complexity of epicardial ablations has limited the technique to high volume clinical centers. Existing tools to map and characterize coronary arteries for epicardial ablation are largely limited in utility due to complexity and inaccuracy and include, for example, catheter electrograms, integration of three-dimensional (3D) images, and fluoroscopy.

Systems for displaying epicardial vascular information are known from US-2017/372474-A1, US-8326419-B2, US-2015/223726-A1, US-2011/201915-A1, and US-2017/303816-A1.

### SUMMARY

In an Example 1, a system for facilitating display of cardiac information associated with a heart of a patient comprises: a display device configured to present an epicardial vascular map, the epicardial vascular map comprising an epicardial cardiac map annotated with a representation of an epicardial vascular structure; and a processing unit configured to: receive a plurality of electrical signals obtained from at least one of a vascular mapping catheter and a magnetically tracked catheter, wherein the vascular mapping catheter comprises one or more electrodes, and wherein the magnetically tracked catheter comprises one or more additional electrodes; determine, from the electrical signals, a plurality of impedance measurements associated with the one or more electrodes of the vascular mapping catheter; access a field map, the field map comprising expected impedance measurements determined based on determined positions of the one or more additional electrodes of the magnetically tracked catheter; determine, based on the plurality of impedance measurements and the field map, a plurality of positions of the one or more electrodes of the vascular mapping catheter; generate, based on the plurality of positions of the one or more electrodes of the vascular mapping catheter, the epicardial vascular structure; access the epicardial cardiac map; annotate the epicardial cardiac map with the representation of the epicardial vascular structure to generate the epicardial vascular map; and facilitate display, via the display device, of the epicardial vascular map.

In an Example 2, the system of Example 2, further comprising the vascular mapping catheter, wherein the vascular mapping catheter is configured to be inserted into a blood vessel associated with the heart.

In an Example 3, the system of either of Examples 1 or 2, further comprising: the magnetically tracked catheter, wherein the magnetically tracked catheter is configured to be inserted into the patient's body; and a tracking system configured to determine positions, within the patient's body, of the one or more additional electrodes of the magnetically tracked catheter.

In an Example 4, the system of any of Examples 1-3, wherein the vascular mapping catheter comprises a guidewire.

In an Example 5, the system of Example 4, wherein the one or more electrodes of the guidewire comprise a portion of the guidewire.

In an Example 6, the system of any of Examples 1-5, wherein the tracking system is further configured to magnetically track positions of the vascular mapping catheter.

In an Example 7, the system of any of Examples 1-6, wherein the plurality of electrical signals are obtained by the one or more electrodes of the vascular mapping catheter, and wherein the plurality of electrical signals correspond to an energy field generated by at least one of the magnetically tracked catheter and a field generator.

In an Example 8, the system of Example 7, wherein the field generator comprises one or more patches disposed external to the patient's body.

In an Example 9, the system of any of Examples 1-6, wherein the plurality of electrical signals are obtained by the one or more additional electrodes of the magnetically tracked catheter, and wherein the plurality of electrical signals correspond to an energy field generated by the vascular mapping catheter.

In an Example 10, a method of facilitating display of epicardial vascular information associated with a heart of a subject comprises receiving a plurality of electrical signals obtained from at least one of a vascular mapping catheter and a magnetically tracked catheter, wherein the vascular mapping catheter comprises one or more electrodes, and wherein the magnetically tracked catheter comprises one or more additional electrodes; determining, from the electrical signals, a plurality of impedance measurements associated with the one or more electrodes of the vascular mapping catheter; accessing a field map, the field map comprising expected impedance measurements determined based on determined positions of the one or more additional electrodes of the magnetically tracked catheter; determining, based on the plurality of impedance measurements and the field map, a plurality of positions of the one or more electrodes of the vascular mapping catheter; generating, based on the plurality of positions of the one or more electrodes of the vascular mapping catheter, the epicardial vascular structure; accessing the epicardial cardiac map; annotating the epicardial cardiac map with the representation of the epicardial vascular structure to generate the epicardial vascular map; and facilitating display, via a display device, of the epicardial vascular map.

In an Example 11, the method of Example 10, wherein the vascular mapping catheter comprises a guidewire.

In an Example 12, the method of Example 11, wherein the one or more electrodes of the guidewire comprise a portion of the guidewire.

In an Example 13, the method of any of Examples 10-12, wherein the plurality of electrical signals are obtained by the one or more electrodes of the vascular mapping catheter, and wherein the plurality of electrical signals correspond to an energy field generated by at least one of the magnetically tracked catheter and a field generator.

In an Example 14, the method of any of Examples 10-12, wherein the plurality of electrical signals are obtained by the one or more additional electrodes of the magnetically tracked catheter, and wherein the plurality of electrical signals correspond to an energy field generated by the vascular mapping catheter.

In an Example 15, the method of any of Examples 10-14, further comprising generating, using the magnetically tracked catheter, the epicardial cardiac map.

In an Example 16, a system for facilitating display of cardiac information associated with a heart of a patient comprises: a display device configured to present an epicardial vascular map, the epicardial vascular map comprising an epicardial cardiac map annotated with a representation of an epicardial vascular structure; and a processing unit configured to: receive a plurality of electrical signals obtained from at least one of a vascular mapping catheter and a magnetically tracked catheter, wherein the vascular mapping catheter comprises one or more electrodes, and wherein the magnetically tracked catheter comprises one or more additional electrodes; determine, from the electrical signals, a plurality of impedance measurements associated with the one or more electrodes of the vascular mapping catheter; access a field map, the field map comprising expected impedance measurements determined based on determined positions of the one or more additional electrodes of the magnetically tracked catheter; determine, based on the plurality of impedance measurements and the field map, a plurality of positions of the one or more electrodes of the vascular mapping catheter; generate, based on the plurality of positions of the one or more electrodes of the vascular mapping catheter, the epicardial vascular structure; access the epicardial cardiac map; annotate the epicardial cardiac map with the representation of the epicardial vascular structure to generate the epicardial vascular map; and facilitate display, via the display device, of the epicardial vascular map.

In an Example 17, the system of Example 16, further comprising the vascular mapping catheter, wherein the vascular mapping catheter is configured to be inserted into a blood vessel associated with the heart.

In an Example 18, the system of Example 16, further comprising: the magnetically tracked catheter, wherein the magnetically tracked catheter is configured to be inserted into the patient's body; and a tracking system configured to determine positions, within the patient's body, of the one or more additional electrodes of the magnetically tracked catheter.

In an Example 19, the system of Example 16, wherein the vascular mapping catheter comprises a guidewire.

In an Example 20, the system of Example 19, wherein the processing unit is further configured to determine a diameter of a blood vessel corresponding to the epicardial vascular structure.

In an Example 21, the system of Example 16, wherein the tracking system is further configured to magnetically track positions of the vascular mapping catheter.

In an Example 22, the system of Example 16, wherein the plurality of electrical signals are obtained by the one or more electrodes of the vascular mapping catheter, and wherein the plurality of electrical signals correspond to an energy field generated by at least one of the magnetically tracked catheter and a field generator.

In an Example 23, the system of Example 22, wherein the field generator comprises one or more patches disposed external to the patient's body.

In an Example 24, the system of Example 16, wherein the plurality of electrical signals are obtained by the one or more additional electrodes of the magnetically tracked catheter, and wherein the plurality of electrical signals correspond to an energy field generated by the vascular mapping catheter.

In an Example 25, a system for facilitating display of cardiac information associated with a heart of a patient comprises: a vascular mapping catheter configured to be inserted into a blood vessel associated with the heart and comprising one or more electrodes; a magnetically tracked catheter configured to be inserted into the patient's body and comprising one or more additional electrodes; a tracking system configured to determine positions, within the patient's body, of the one or more additional electrodes; a display device configured to present an epicardial vascular map, the epicardial vascular map comprising an epicardial cardiac map annotated with a representation of an epicardial vascular structure; and a processing unit configured to: receive a plurality of electrical signals from at least one of the vascular mapping catheter and the magnetically tracked catheter; determine, from the electrical signals, a plurality of impedance measurements associated with the one or more electrodes of the vascular mapping catheter; access a field map, the field map comprising expected impedance measurements determined based on the determined positions of the one or more additional electrodes; determine, based on the plurality of impedance measurements and the field map, a plurality of positions, of the one or more electrodes of the vascular mapping catheter; generate, based on the plurality of positions of the vascular mapping catheter, the epicardial vascular structure; access the epicardial cardiac map; annotate the epicardial cardiac map with the representation of the epicardial vascular structure to generate the epicardial vascular map; and facilitate display, via the display device, of the epicardial vascular map.

In an Example 26, the system of Example 25, wherein the vascular mapping catheter comprises a guidewire.

In an Example 27, the system of Example 26, wherein the one or more electrodes of the guidewire comprise a portion of the guidewire.

In an Example 28, the system of Example 25, wherein the tracking system is further configured to magnetically track positions of the vascular mapping catheter.

In an Example 29, the system of Example 25, wherein the plurality of electrical signals are obtained by the one or more electrodes of the vascular mapping catheter, and wherein the plurality of electrical signals correspond to an energy field generated by at least one of the magnetically tracked catheter and a field generator.

In an Example 30, the system of Example 29, wherein the field generator comprises one or more patches disposed external to the patient's body.

In an Example 31, the system of Example 25, wherein the plurality of electrical signals are obtained by the one or more additional electrodes of the magnetically tracked catheter, and wherein the plurality of electrical signals correspond to an energy field generated by the vascular mapping catheter.

In an Example 32, a method of facilitating display of epicardial vascular information associated with a heart of a subject comprises: receiving a plurality of electrical signals obtained from at least one of a vascular mapping catheter and a magnetically tracked catheter, wherein the vascular mapping catheter comprises one or more electrodes, and wherein the magnetically tracked catheter comprises one or more additional electrodes; determining, from the electrical signals, a plurality of impedance measurements associated with the one or more electrodes of the vascular mapping catheter; accessing a field map, the field map comprising expected impedance measurements determined based on determined positions of the one or more additional electrodes of the magnetically tracked catheter; determining, based on the plurality of impedance measurements and the field map, a plurality of positions of the one or more electrodes of the vascular mapping catheter; generating, based on the plurality of positions of the one or more electrodes of the vascular mapping catheter, the epicardial vascular structure; accessing the epicardial cardiac map; annotating the epicardial cardiac map with the representation of the epicardial vascular structure to generate the epicardial vascular map; and facilitating display, via a display device, of the epicardial vascular map.

In an Example 33, the method of Example 32, wherein the vascular mapping catheter comprises a guidewire.

In an Example 34, the method of Example 32, wherein the plurality of electrical signals are obtained by the one or more electrodes of the vascular mapping catheter, and wherein the plurality of electrical signals correspond to an energy field generated by at least one of the magnetically tracked catheter and a field generator.

In an Example 35, the method of Example 32, wherein the plurality of electrical signals are obtained by the one or more additional electrodes of the magnetically tracked catheter, and wherein the plurality of electrical signals correspond to an energy field generated by the vascular mapping catheter.

While multiple embodiments are disclosed, still other embodiments of the presently disclosed subject matter will become apparent to those skilled in the art from the following detailed description, which shows and describes illustrative embodiments of the disclosed subject matter. Accordingly, the drawings and detailed description are to be regarded as illustrative in nature and not restrictive.

### BRIEF DESCRIPTION OF THE DRAWINGS

FIG. 1 is a conceptual schematic diagram depicting an illustrative cardiac mapping system, in accordance with embodiments of the subject matter disclosed herein.
FIG. 2 is a block diagram depicting an illustrative processing unit, in accordance with embodiments of the subject matter disclosed herein.
FIGS. 3A and 3B depict an illustrative epicardial vascular map representing a portion of a patient's heart, in accordance with embodiments of the subject matter disclosed herein.
FIG. 4 is a flow diagram depicting an illustrative method for providing an epicardial vascular map, in accordance with embodiments of the subject matter disclosed herein.

As the terms are used herein with respect to measurements (e.g., dimensions, characteristics, attributes, components, etc.), and ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

Although the term "block" may be used herein to connote different elements illustratively employed, the term should not be interpreted as implying any requirement of, or particular order among or between, various blocks disclosed herein. Similarly, although illustrative methods may be represented by one or more drawings (e.g., flow diagrams, communication flows, etc.), the drawings should not be interpreted as implying any requirement of, or particular order among or between, various steps disclosed herein. However, certain embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

As used herein, the term "based on" is not meant to be restrictive, but rather indicates that a determination, identification, prediction, calculation, and/or the like, is performed by using, at least, the term following "based on" as an input. For example, predicting an outcome based on a particular piece of information may additionally, or alternatively, base the same determination on another piece of information.

### DETAILED DESCRIPTION

Embodiments of systems and methods described herein facilitate processing sensed cardiac electrical signals to present, via a graphical user interface (GUI) presented via a display device, an epicardial vascular map. In embodiments, an epicardial vascular map refers to a visual representation of one or more aspects of an epicardial vessel (e.g., a vessel disposed on or adjacent to the epicardium of a patient's heart) such as, for example, an epicardial vascular structure (e.g., a visual geometric electroanatomical model of one or more epicardial vessels), which may be displayed with (e.g., annotated on) an epicardial cardiac map (e.g., a visual geometric electroanatomical model of an epicardium surface). In embodiments, one or more characteristics of the one or more vessels may be determined and representations thereof may also, or alternatively, be presented via the GUI.

According to embodiments, to perform aspects of embodiments of the methods described herein, cardiac electrical signals may be obtained from a mapping catheter (e.g., associated with a mapping system), a recording system, a coronary sinus (CS) catheter or other reference catheter (which may, in embodiments, be a vascular mapping catheter), an ablation catheter, a memory device (e.g., a local memory, a cloud server, etc.), a communication component, a medical device (e.g., an implantable medical device, an external medical device, a telemetry device, etc.), and/or the like.

As the term is used herein, a sensed cardiac electrical signal may refer to one or more sensed signals. Each cardiac electrical signal may include a number of intracardiac electrograms (EGMs) sensed within a patient's heart, and may include any number of features that may be ascertained by aspects of the system 100. Examples of cardiac electrical signal features include, but are not limited to, impedance measurements, activation times, activations, activation waveforms, filtered activation waveforms, minimum voltage values, maximum voltages values, maximum negative time-derivatives of voltages, instantaneous potentials, voltage amplitudes, dominant frequencies, peak-to-peak voltages, and/or the like. A cardiac electrical signal feature may refer to one or more features extracted from one or more cardiac electrical signals, derived from one or more features that are extracted from one or more cardiac electrical signals, and/or the like. Additionally, a representation, on a cardiac and/or a surface map, of a cardiac electrical signal feature may represent one or more cardiac electrical signal features, an interpolation of a number of cardiac electrical signal features, and/or the like.

Each cardiac signal also may be associated with a set of respective position coordinates that corresponds to the location at which the cardiac electrical signal was sensed. Each of the respective position coordinates for the sensed cardiac signals may include three-dimensional Cartesian coordinates, polar coordinates, and/or the like. In embodiments, other coordinate systems can be used. In embodiments, an arbitrary origin is used and the respective position coordinates refer to positions in space relative to the arbitrary origin. Since, in embodiments, the cardiac signals may be sensed on the cardiac surfaces, the respective position coordinates may be on the endocardial surface, epicardial surface, in the mid-myocardium of the patient's heart, within or on a vessel surface, and/or in the vicinity of one of one of these.

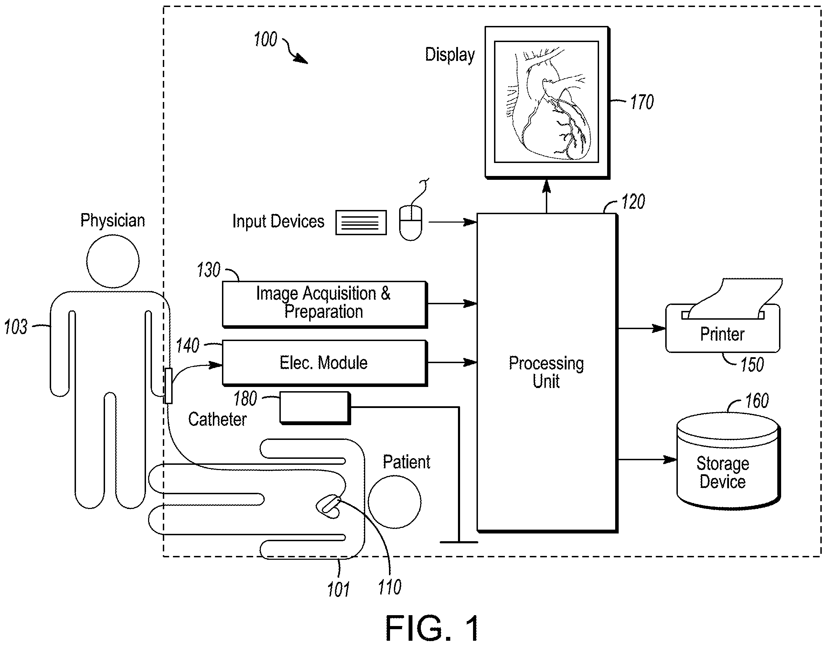

FIG. 1 shows a schematic diagram of an exemplary embodiment of a cardiac mapping system 100. As indicated above, embodiments of the subject matter disclosed herein may be implemented in a mapping system (e.g., the mapping system 100), while other embodiments may be implemented in an ablation system, a recording system, a computer analysis system, and/or the like. The mapping system 100 includes a moveable catheter 110. The catheter 110 may be, be similar to, include, or be included within, a magnetically tracked catheter, a vascular mapping catheter, a guidewire, an ablation catheter, and/or the like. Additionally, the catheter 110 may represent any number of different catheters such as for example, a pair of catheters (one magnetically tracked catheter and one vascular mapping catheter, two magnetically tracked catheters, one or more magnetically tracked vascular mapping catheters, etc.).

The catheter 110 may include one or more electrodes. For example, in embodiments, the catheter 110 may include multiple spatially distributed electrodes. During a signal-acquisition stage of a cardiac mapping procedure, the catheter 110 may be displaced to multiple locations within the heart chamber, and/or the chamber surrounding the heart, into which the catheter 110 is inserted. In embodiments, the catheter 110 may include one electrode, which may, for example, be a portion of the catheter 110 itself (e.g., in implementations in which a guidewire is used as a vascular mapping catheter). In embodiments the distal end of the catheter 110 may be fitted with multiple electrodes spread somewhat uniformly over the catheter. For example, the electrodes may be mounted on the catheter 110 following a 3D olive shape, a basket shape, and/or the like. The electrodes may be mounted on a device capable of deploying the electrodes into the desired shape while inside the heart, and retracting the electrodes when the catheter is removed from the heart. To allow deployment into a 3D shape in the heart, electrodes may be mounted on a balloon, shape memory material such as Nitinol, actuable hinged structure, and/or the like.

According to embodiments, the catheter 110 may be a mapping catheter, an ablation catheter, a diagnostic catheter, a CS catheter, and/or the like. Illustrative examples of such catheters are described, for example, in U.S. Patent No. 9,848,795, issued December 26, 2017 to Boston Scientific Scimed, Inc., of Maple Grove, Minnesota. For example, aspects of embodiments of the catheter 110, the electrical signals obtained using the catheter 110, and subsequent processing of the electrical signals, as described herein, may also be applicable in implementations having a recording system, ablation system, and/or any other system having a catheter with electrodes that may be configured to obtain cardiac electrical signals.

At each of the locations to which the catheter 110 is moved, the catheter's multiple electrodes acquire signals resulting from the electrical activity in the heart. Consequently, reconstructing and presenting to a user (such as a doctor and/or technician) physiological data pertaining to the heart's electrical activity may be based on information acquired at multiple locations, thereby providing a more accurate and faithful reconstruction of physiological behavior of the epicardium surface. The acquisition of signals at multiple catheter locations in the heart chamber, or chamber surrounding the heart, enables the catheter to effectively act as a "mega-catheter" whose effective number of electrodes and electrode span is proportional to the product of the number of locations in which signal acquisition is performed and the number of electrodes the catheter has.

To enhance the quality of the reconstructed physiological information at the epicardium surface, in some embodiments the catheter 110 is moved to more than three locations (for example, more than 5, 10, or even 50 locations). Additionally, in some embodiments the reconstructed physiological information is computed based on signals measured over several heart beats, either at a single catheter location or over several locations. In circumstances where the reconstructed physiological information is based on multiple measurements over several heart beats, the measurements may be synchronized with one another so that the measurement are performed at approximately the same phase of the heart cycle. The signal measurements over multiple beats may be synchronized based on features detected from physiological data such as surface electrocardiograms (ECGs) and/or intracardiac electrograms (EGMs).

The cardiac mapping system 100 further includes a processing unit 120 which performs several of the operations pertaining to the mapping procedure, including the reconstruction procedure to determine the physiological information at the epicardium surface (e.g., as described above) and/or within a heart chamber and/or a chamber surrounding the heart. The processing unit 120 also may perform a catheter registration procedure. The processing unit 120 also may generate a 3D grid used to aggregate the information captured by the catheter 110 and to facilitate display of portions of that information.

The location of the catheter 110 inserted into the patient's body can be determined using a conventional sensing and tracking system 180 that provides the 3D spatial coordinates of the catheter and/or its electrodes with respect to the catheter's coordinate system as established by the sensing and tracking system. These 3D spatial locations may be used in building a 3D grid. Embodiments of the system 100 may use a hybrid location technology that combines impedance location with magnetic location technology. This combination may enable the system 100 to accurately track catheters that are connected to the system 100. Magnetic location technology uses magnetic fields generated by a localization generator positioned under the patient table to track catheters with magnetic sensors. Impedance location technology may be used to track catheters that may not be equipped with a magnetic location sensor, and may utilize surface ECG patches.

In embodiments, to perform a mapping procedure and reconstruct physiological information on the epicardium surface, the processing unit 120 may align the coordinate system of the catheter 110 with the epicardium surface's coordinate system. The processing unit 110 (or some other processing component of the system 100) may determine a coordinate system transformation function that transforms the 3D spatial coordinates of the catheter's locations into coordinates expressed in terms of the epicardium surface's coordinate system, and/or vice-versa. In embodiments, such a transformation may not be necessary, as embodiments of the 3D grid described herein may be used to capture contact and non-contact EGMs, and select mapping values based on statistical distributions associated with nodes of the 3D grid. The processing unit 120 also may perform post-processing operations on the physiological information to extract and display useful features of the information to the operator of the system 100 and/or other persons (e.g., a physician).

According to embodiments, the signals acquired by the electrode(s) of catheter 110 are passed to the processing unit 120 via an electrical module 140, which may include, for example, a signal conditioning component. The electrical module 140 may be configured to receive the signals communicated from the catheter 110 and perform signal enhancement operations on the signals before they are forwarded to the processing unit 120. The electrical module 140 may include signal conditioning hardware, software, and/or firmware that may be used to amplify, filter and/or sample intracardiac potential measured by one or more electrodes. The intracardiac signals typically have a maximum amplitude of 60mV, with a mean of a few millivolts.

In some embodiments the signals are bandpass filtered in a frequency range (e.g., 0.5-500Hz) and sampled with analog to digital converters (e.g., with 15-bit resolution at 1kHz). To avoid interference with electrical equipment in the room, the signal may be filtered to remove the frequency corresponding to the power supply (e.g., 60 Hz). Other types of signal processing operations such as spectral equalization, automatic gain control, etc. may also take place. For example, in embodiments, the intracardiac signals may be unipolar signals, measured relative to a reference (which may be a virtual reference) such as, for example, a coronary sinus catheter or Wilson's Central Terminal (WCT), from which the signal processing operations may compute differences to generate multipolar signals (e.g., bipolar signals, tripolar signals, etc.). The signals may be otherwise processed (e.g., filtered, sampled, etc.) before and/or after generating the multipolar signals. The resultant processed signals are forwarded by the module 140 to the processing unit 120 for further processing.

In embodiments, the processing unit 120 may be configured to process the resultant processed signals. In embodiments, because the processing unit 120 may be configured to process any number of different types of electrical signals, whether they have been preprocessed or not, the terms "electrical signal(s)," "cardiac electrical signal(s)" and terms including one or more of the aforementioned, shall be understood to refer to electrical signals, processed (e.g., "pre-processed") electrical signals, raw signal data, interpolated electrical signals, estimated electrical signals, and/or any other type of information representing an electrical signal, as described herein. In embodiments, the processing unit 120 may be configured to facilitate processing sensed cardiac electrical signals to present, via a GUI, representations of an epicardial vascular structure associated with an epicaridal cardiac map.

Embodiments of the processing unit 120 may be configured to receive a number of electrical signals such as, for example, cardiac electrical signals (e.g., electrograms). The processing unit 120 may receive the electrical signals from the electrical module 140, from a memory device, from a catheter (e.g., the catheter 110), from another computing device, from a user via a user input device, and/or the like. In embodiments, the processing unit 120 may receive an indication of a measurement location corresponding to each electrical signal. The processing unit 120 may be configured to generate, based on the electrical signals, a cardiac map (e.g., an epicardial vascular map), which may be presented via a display device 170. In embodiments, the cardiac map includes a number of annotations representing a number of cardiac signal features, which may include, for example, one or more epicaridal vascular structures, vascular characteristics, impedance measurements, activation times, minimum voltage values, maximum voltage values, maximum negative time-derivatives of voltage, instantaneous potentials, voltage amplitudes, dominant frequencies, and/or peak-to-peak voltages.

In embodiments, for example, the processing unit 120 may be configured to receive electrical signals obtained from at least one of a vascular mapping catheter and a magnetically tracked catheter, where the vascular mapping catheter comprises one or more electrodes, and where the magnetically tracked catheter comprises one or more additional electrodes; and determine, from the electrical signals, impedance measurements associated with the one or more electrodes of the vascular mapping catheter. The processing unit 120 may be further configured to access a field map, the field map including expected impedance measurements determined based on determined positions of the one or more additional electrodes of the magnetically tracked catheter; and to determine, based on the impedance measurements and the field map, a number of positions of the one or more electrodes of the vascular mapping catheter.

According to embodiments, the processing unit 120 may be further configured to generate, based on the positions of the one or more electrodes of the vascular mapping catheter, an epicardial vascular structure; access (and/or generate) an epicardial cardiac map; and annotate the epicardial cardiac map with the representation of the epicardial vascular structure to generate an epicardial vascular map. In embodiments, the processing unit may be configured to facilitate display, via a display device 170, of the epicardial vascular map.

As further shown in FIG. 1, the cardiac mapping system 100 also may include peripheral devices such as a printer 150 and/or display device 170, both of which may be interconnected to the processing unit 120. Additionally, the mapping system 100 includes storage device 160 that may be used to store data acquired by the various interconnected modules, including the volumetric images, raw data measured by electrodes and/or the resultant endocardium representation computed therefrom, the partially computed transformations used to expedite the mapping procedures, the reconstructed physiological information corresponding to the epicardium surface, and/or the like.

In embodiments, the processing unit 120 may be configured to automatically improve the accuracy of its algorithms by using one or more artificial intelligence (i.e., machine-learning) techniques, classifiers, and/or the like. In embodiments, for example, the processing unit may use one or more supervised and/or unsupervised techniques such as, for example, support vector machines (SVMs), k-nearest neighbor techniques, artificial neural networks, and/or the like. In embodiments, classifiers may be trained and/or adapted using feedback information from a user, other metrics, and/or the like.

The illustrative cardiac mapping system 100 shown in FIG. 1 is not intended to suggest any limitation as to the scope of use or functionality of embodiments of the present disclosure. The illustrative cardiac mapping system 100 also should not be interpreted as having any dependency or requirement related to any single component or combination of components illustrated therein. Additionally, various components depicted in FIG. 1 may be, in embodiments, integrated with various ones of the other components depicted therein (and/or components not illustrated), all of which are considered to be within the ambit of the subject matter disclosed herein. For example, the electrical module 140 may be integrated with the processing unit 120. Additionally, or alternatively, aspects of embodiments of the cardiac mapping system 100 may be implemented in a computer analysis system configured to receive cardiac electrical signals and/or other information from a memory device (e.g., a cloud server, a mapping system memory, etc.), and perform aspects of embodiments of the methods described herein for processing cardiac information (e.g., generating epicardial vascular structures, etc.). That is, for example, a computer analysis system may include a processing unit 120, but not a mapping catheter.

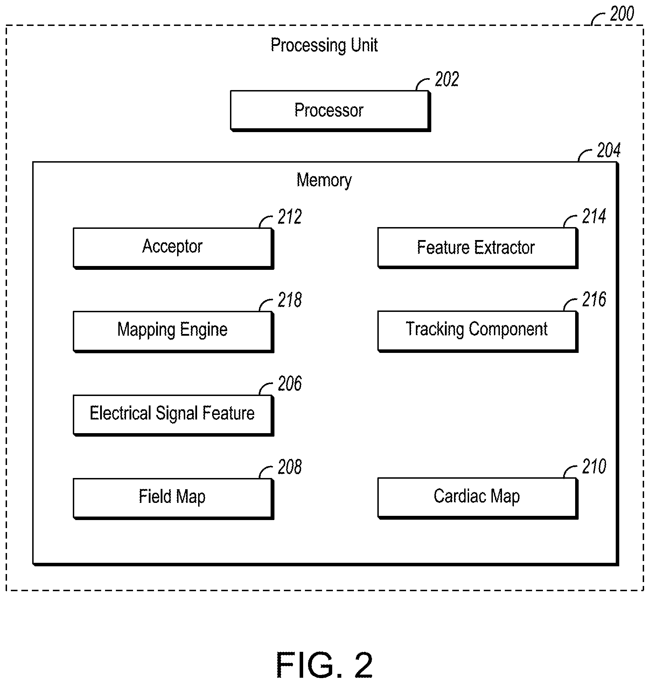

FIG. 2 is a block diagram of an illustrative processing unit 200, in accordance with embodiments of the disclosure. The processing unit 200 may be, be similar to, include, or be included in the processing unit 120 depicted in FIG. 1. As shown in FIG. 2, the processing unit 200 may be implemented on a computing device that includes a processor 202 and a memory 204. Although the processing unit 200 is referred to herein in the singular, the processing unit 200 may be implemented in multiple instances (e.g., as a server cluster), distributed across multiple computing devices, instantiated within multiple virtual machines, and/or the like. One or more components for facilitating cardiac mapping may be stored in the memory 204. In embodiments, the processor 202 may be configured to instantiate the one or more components to process electrical signals received from electrodes, extract one or more electrical signal features 206 (e.g., impedance measurements) from one or more electrical signals, and to generate one or more field maps 208 and/or epicardial cardiac maps 210, either of which may be stored in the memory 204.

As is further depicted in FIG. 2, the processing unit 200 may include an acceptor 212 configured to receive electrical signals. The acceptor 212 may be configured to receive electrical signals from a catheter (e.g., the mapping catheter 110 depicted in FIG. 1), a memory device (e.g., the memory 204), a server, and/or the like. The measured electrical signals may include a number of intracardiac electrograms (EGMs) sensed within a patient's heart, extracardiac electrograms sensed outside of a patient's heart, and/or the like. The acceptor 212 may also receive an indication of a measurement location corresponding to each of the electrical signals. In embodiments, the acceptor 212 may be configured to determine whether to accept the electrical signals that have been received. The acceptor 212 may utilize any number of different components and/or techniques to determine which electrical signals or beats to accept, such as filtering, beat matching, morphology analysis, positional information (e.g., catheter motion), respiration gating, and/or the like.

The accepted electrical signals are received by a feature extractor 214 that is configured to extract at least one electrical signal feature from each of the electrical signals. In embodiments, an extracted electrical signal feature may be used to annotate a cardiac map, in which case, the extracted electrical signal feature may be referred to, interchangeably, as an annotation feature. In embodiments in which the electrical signal is a cardiac electrical signal, an extracted signal feature may be referred to, interchangeably, as a cardiac electrical signal feature. In embodiments, the at least one electrical signal feature includes at least one value corresponding to at least one annotation metric. The at least one feature may include at least one event, where the at least one event includes the at least one value corresponding to the at least one metric and/or at least one corresponding time (a corresponding time does not necessarily exist for each annotation feature).

According to embodiments, the at least one electrical signal feature may include, for example, an activation time, detected activation (e.g., a component of an activation waveform), activation waveform, activation histogram, minimum voltage value, maximum voltage value, maximum negative time-derivative of voltage, an instantaneous potential, a voltage amplitude, a dominant frequency, a peak-to-peak voltage, an activation duration, an annotation waveform (e.g., an activation waveform), an impedance measurement, an epicardial vascual structure, a vascular characteristic, and/or the like. A cardiac electrical signal feature may refer to one or more features extracted from one or more cardiac electrical signals, derived from one or more features that are extracted from one or more cardiac electrical signals, and/or the like. Additionally, a representation, on a cardiac and/or a surface map, of a cardiac electrical signal feature may represent one or more cardiac electrical signal features, an interpolation of a number of cardiac electrical signal features, and/or the like.

As shown in FIG. 2, the processing unit 200 includes a tracking component 216. According to embodiments, the tracking component 216 is configured to facilitate tracking locations of one or more catheters. The tracking component 216 may be, be similar to, include, be included within, or otherwise correspond to the tracking system 180. Illustrative examples of the tracking component 216 may include, for example, one or more aspects of the tracking systems disclosed in U.S. Patent No. 8,167,876, issued May 1, 2012 to Rhythmia Medical, Inc., of Burlington, Massachusetts.

That is, for example, embodiments disclosed herein include a method and system for determining the position of a catheter in a patient's epicardial vasculature using a pre-determined model of the field (e.g., a field map) that provides expected signal measurements of the field at various locations within the patient's body. For example, embodiments described herein provide a method for tracking electrodes mounted on catheters within and relative to the cardiac cavity, including any number of chambers within this cavity and the blood vessels surrounding it, but it can be used for tracking catheters in other body organs as well. Electrodes can be mounted on one or multiple catheters and by tracking these electrodes the location of such catheters can be determined and the catheters can be tracked. By knowing the physical characteristics of a catheter and the position of the electrodes on it, it is possible to track specific portion of the catheter (e.g., the tip) or to determine the shape and the orientation of the catheter (e.g., by using a spline fitting method on the location of multiple electrodes of the same catheter). Electrodes can also be mounted on other devices that require tracking inside the heart cavity.

In some aspects, the tracking is accomplished by generating a multitude of fields using current injecting electrodes (CIE) positioned and secured in a stable location (e.g., coronary sinus, atrial appendage, apex) and using measurements of the same fields on electrodes mounted on other catheters to locate the electrodes. The purpose of the CIEs is to inject current into the heart cavity and/or the cavity surrounding the heart. For example, each CIE pair can define a source and sink electrode, respectively, for injecting current.

In general, in embodiments, a field mapping catheter that includes one or more potential measuring electrodes (PME) that can measure the fields (e.g., measure potentials in the heart cavity in response to the current provided by the CIEs) and at the same time can be tracked by an independent tracking system is used for generating a field map. The field map provides expected signal measurements of the field at various locations within the heart cavity, cavity surrounding the heart, and/or epicardial vasculature. A field map is an example of a pre-determined model of the field. Other methods for generating a predetermined model of the field exist and can be used. For example, a pre-determined model can be generated based on a volumetric image of the medium (CT or MRI) and an analysis of the medium based on that image to generate a physical model of the fields in the medium.

An independent tracking system can be used in embodiments, and may include any system for tracking catheters inside the body, such as systems based on electric or magnetic signals generated externally and detected by one or more tracking elements, affixed to a catheter, or systems based on electric or magnetic signals generated internally from a catheter and detected by one or more sensors external to the body or internal to the body, affixed to other catheters. Such a system may be based, for example, on tracking electric or magnetic signals generated externally and detected by one or more tracking elements, such as sensors, affixed to a catheter. Additionally or alternatively, tracking elements such as emitters or beacons affixed to the catheter may emit electric or magnetic signatures that are detected by an independent tracking system, and used to determine the location of the emitters, and thus the location and orientation of a catheter. For example, a collection of miniaturized coils oriented to detect orthogonal magnetic fields and forming a sensor may be placed inside the catheter to detect the generated magnetic fields. The independent tracking systems are often disposed outside the patient's body at a distance that enables the system to either generate radiation of suitable strength (i.e., generate signals whose amplitude will not harm the patient or otherwise interfere with the operation of other apparatus disposed in the near vicinity of the sensing and tracking system), or detect magnetic or electric radiation emitted by the emitters affixed to a catheter.

U.S. Patent No. 8,538,509, issued September 17, 2013, describes an alternative independent tracking system utilizing a multi-electrode array (MEA) for generating and sensing fields in the cavity for tracking PME and catheters. The system utilizes reference electrodes secured in stable positions to reference the tracking coordinate system to the organ, compensating for movement of the cavity in space that can result from different reasons such as patient movements or patient respiration.

According to embodiments, as described herein, the tracking component 216 may be configured to generate one or more field maps 208. A field map 208 may include, for example, an impedance field map, which may be generated using impedance measurements obtained from a magnetically tracked catheter, a vascular mapping catheter, and/or the like. In embodiments, illustrative examples of methods of generating field maps are described in U.S. Patent No. 8,167,876, issued May 1, 2012 to Rhythmia Medical, Inc., of Burlington, Massachusetts.

In embodiments, the model of the field is generated using the field measurements and the positions measurements collected by the field mapping catheter. The model can be generated based on physical characteristics of the medium. The model of the field associates the field measurements (e.g., impedance measurements) to each location in space. Once a field map is generated, in embodiments, the independent tracking system can be turned off, any internal element of the system used to generate the field map can be taken out of the body, and the field mapping catheter can also be taken out of the body. However, the CIE used to generate the fields may be left in their stable locations for subsequent use in tracking other electrodes. In embodiments, removing the potential measuring electrodes used to generate the field map can be advantageous when it is desired to have fewer catheters inside the body organ for clinical reasons, or when certain tracking fields interfere with other instruments in the operating room. Using the field map it is possible to determine the location of any electrodes that can measure the generated fields (e.g., the fields generated using the current injecting electrodes) inside the volume covered by the field map. The position of a tracked electrode is determined by comparing the measured field value and the modeled field values. The position in the field map that holds a value matching the measurement of the tracked electrode is assigned as the location of that electrode.

In some embodiments, potentials measured in response to the injected current (e.g., tracking signals) can be used to continuously monitor the position of one or more catheters in the heart cavity, cavity surrounding the heart, and/or epicardial vasculature, even as the catheters are moved therein.

In some aspects, the system tracks electrodes inside a body without having these electrodes injecting currents or emitting any field that needs to be detected. Rather, the fields can be generated by CIE positioned at fixed locations relative to the organ (e.g., external patches, electrodes on a catheter, etc.). This allows tracking of a large number of electrodes simultaneously, as the tracked electrodes are not polled one after the other as is the case with some other tracking methods.

In some additional aspects, the system does not require any external patches to be attached to the body, or any other external energy emitter. In some embodiments, the system only uses internal electrodes to inject currents. Furthermore, the method does not require any knowledge about the location in space of the current injecting electrodes. In some embodiments, the CIE can be positioned such that the current injection is taking place from objects that are secured to the heart itself, reducing inaccuracies from motion artifacts that are experienced by systems that are referenced to an external coordinate system.

According to embodiments, the electrical signals may be obtained by one or more electrodes of a vascular mapping catheter, and may correspond to an energy field generated by at least one of the magnetically tracked catheter and a field generator. The field generator may include one or more catheters, one or more external patches, and/or the like. In embodiments, the electrical signals may be obtained by one or more additional electrodes of the magnetically tracked catheter, and may correspond to an energy field generated by the vascular mapping catheter. That is, for example, an electrically-active guidewire may be used as a vascular mapping catheter, and may be configured to inject, via one or more electrodes, a current that is sunk at one or more electrodes of the magnetically tracked catheter. In either case, the one or more electrodes of the vascular mapping catheter may include independent electrodes coupled to a portion of the vascular mapping catheter, portions of the catheter itself (e.g., the conductive metal portion of a guidewire), and/or the like.

Additionally, the processing unit 200 includes a mapping engine 218 that is configured to facilitate presentation of an epicardial map 210 corresponding to a cardiac surface based on the electrical signals. In embodiments, the map 210 may include a voltage map, an activation map, a fractionation map, velocity map, confidence map, an impedance map, and/or the like. In embodiments, the mapping engine 218 may be, include, be similar to, be included within, and/or be otherwise integrated with the tracking component 216. In embodiments, the mapping engine 218 may be configured to facilitate display, via the display device, of the cardiac map and/or representations of electrical signals.

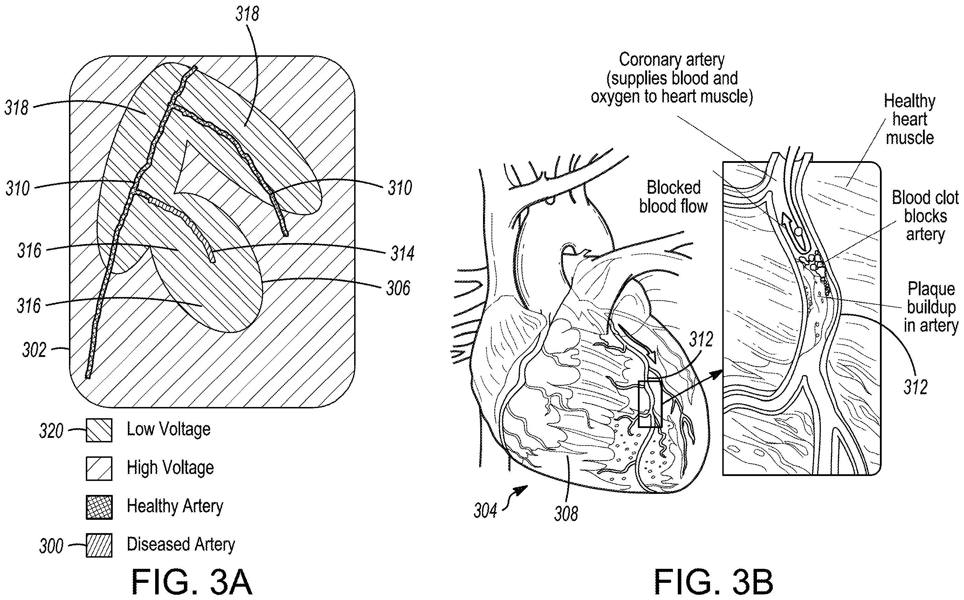

For example, FIG. 3A depicts an illustrative GUI 300 configured to present an epicardial cardiac map 302 representative of information associated with a patient's heart 304 (depicted in FIG. 3B), in accordance with embodiments of the subject matter disclosed herein. According to embodiments, the map 302 includes an epicardial cardiac map 306 that represents an epicardium surface 308 of the heart 304. The map 302 further includes one or more representations 310 of one or more epicardial vascular structures generated to represent one or more epicardial vessels 312 (e.g., a coronary artery, etc.). The cardiac map 302 may further include annotations 314 that are representations of one or more vascular characteristics.

As shown, for example, the cardiac map 302 includes a portion of the representation 310 of the vascular structure displayed in a different color than the other portions thereof. This difference in color may represent, for example, ischemia, which may correspond to a representation 316 of dead heart tissue, enabling a clinician to distinguish between, for example, a diseased structure and a fatty structure 318 that may, for example, both be represented as having a similar voltage. As shown in FIG. 3A, the cardiac map may include annotations 320 such as, for example, color differences, to represent different ranges of annotation values (e.g., voltages, impedance levels, etc.).

The illustrative processing unit 200 shown in FIG. 2 and the illustrative GUI 300 are not intended to suggest any limitation as to the scope of use or functionality of embodiments of the present disclosure. Neither should the illustrative processing unit 200 and/or the GUI 300 be interpreted as having any dependency or requirement related to any single component or combination of components illustrated therein. Additionally, any one or more of the components and/or features depicted in FIGS. 2, 3A, and 3B may be, in embodiments, integrated with various ones of the other components and/or features depicted therein (and/or components not illustrated), all of which are considered to be within the ambit of the subject matter disclosed herein.

The processing unit 200 may (alone and/or in combination with other components of the system 100 depicted in FIG. 1, and/or other components not illustrated) perform any number of different functions and/or processes associated with cardiac mapping (e.g., triggering, blanking, field mapping, etc.) such as, for example, those described in U.S. Patent 8,428,700, entitled "ELECTROANATOMICAL MAPPING;" U.S. Patent 8,948,837, entitled "ELECTROANATOMICAL MAPPING;" U.S. Patent 8,615,287, entitled "CATHETER TRACKING AND ENDOCARDIUM REPRESENTATION GENERATION;" U.S. Patent Publication 2015/0065836, entitled "ESTIMATING THE PREVALENCE OF ACTIVATION PATTERNS IN DATA SEGMENTS DURING ELECTROPHYSIOLOGY MAPPING;" U.S. Patent 6,070,094, entitled "SYSTEMS AND METHODS FOR GUIDING MOVABLE ELECTRODE ELEMENTS WITHIN MULTIPLE-ELECTRODE STRUCTURE;" U.S. Patent 6,233,491, entitled "CARDIAC MAPPING AND ABLATION SYSTEMS;" U.S. Patent 6,735,465, entitled "SYSTEMS AND PROCESSES FOR REFINING A REGISTERED MAP OF A BODY CAVITY;".

According to embodiments, various components of the mapping system 100, illustrated in FIG. 1, and/or the processing unit 200, illustrated in FIG. 2, may be implemented on one or more computing devices. A computing device may include any type of computing device suitable for implementing embodiments of the disclosure. Examples of computing devices include specialized computing devices or general-purpose computing devices such "workstations," "servers," "laptops," "desktops," "tablet computers," "hand-held devices," "electronics modules," "processing units," "general-purpose graphics processing units (GPGPUs)," and the like, all of which are contemplated within the scope of FIGS. 1 and 2 with reference to various components of the system 100 and/or processing unit 200.

In embodiments, a computing device includes a bus that, directly and/or indirectly, couples the following devices: a processor, a memory, an input/output (I/O) port, an I/O component, and a power supply. Any number of additional components, different components, and/or combinations of components may also be included in the computing device. The bus represents what may be one or more busses (such as, for example, an address bus, data bus, or combination thereof). Similarly, in embodiments, the computing device may include a number of processors, a number of memory components, a number of I/O ports, a number of I/O components, and/or a number of power supplies. Additionally any number of these components, or combinations thereof, may be distributed and/or duplicated across a number of computing devices.

In embodiments, memory (e.g., the storage device 160 depicted in FIG. 1, and/or the memory 204 depicted in FIG. 2) includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory 160 and/or 204 stores computer-executable instructions for causing a processor (e.g., the processing unit 120 depicted in FIG. 1 and/or the processor 202 depicted in FIG. 2) to implement aspects of embodiments of system components discussed herein and/or to perform aspects of embodiments of methods and procedures discussed herein.

Computer-executable instructions may include, for example, computer code, machine-useable instructions, and the like such as, for example, program components capable of being executed by one or more processors associated with a computing device. Examples of such program components include the electrical signal (electrogram 208), electrical signal feature 206, the map 210, the acceptor 212, the feature extractor 214, the tracking component 216, and/or the mapping engine 218. Program components may be programmed using any number of different programming environments, including various languages, development kits, frameworks, and/or the like. Some or all of the functionality contemplated herein may also, or alternatively, be implemented in hardware and/or firmware.

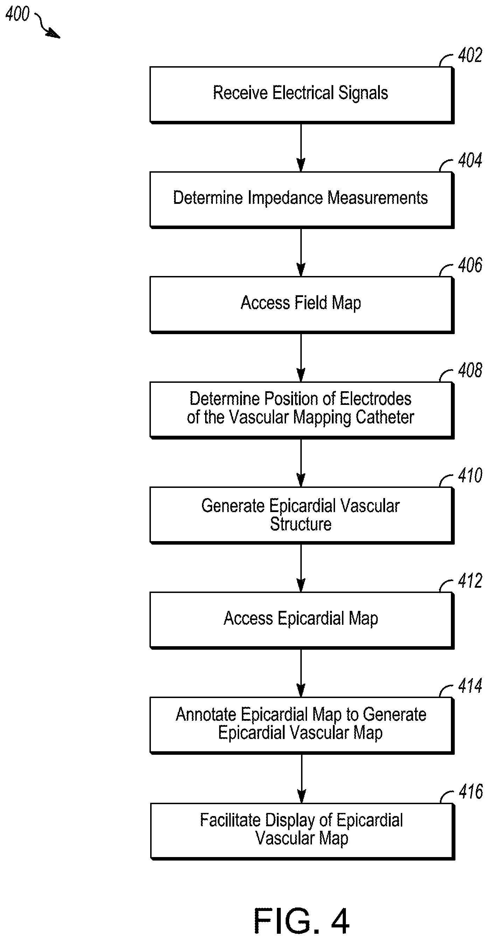

FIG. 4 is a flow diagram depicting an illustrative method 400 of presenting an epicardial vascular map, in accordance with embodiments of the disclosure. Aspects of embodiments of the method 400 may be performed, for example, by a processing unit (e.g., the processing unit 120 depicted in FIG. 1, and/or the processing unit 200 depicted in FIG. 2). Embodiments of the method 400 include receiving a plurality of electrical signals (block 402). The electrical signals may be received from a catheter, a memory device, a computing device, and/or the like. Each of the electrical signals may be, include, be similar to, or be included in an electrogram and may be obtained using one or more catheters such as, for example, a magnetically tracked catheter, a vascular mapping catheter, and/or the like. The catheter may be any catheter having one or more electrodes configured to obtain electrical signals (e.g., the mapping catheter 110 depicted in FIG. 1, a CS catheter, an ablation catheter, a guidewire, etc.).

According to embodiments, the electrical signals may be obtained by one or more electrodes of a vascular mapping catheter, and may correspond to an energy field generated by at least one of the magnetically tracked catheter and a field generator. The field generator may include one or more catheters, one or more external patches, and/or the like. In embodiments, the electrical signals may be obtained by one or more additional electrodes of the magnetically tracked catheter, and may correspond to an energy field generated by the vascular mapping catheter. That is, for example, an electrically-active guidewire may be used as a vascular mapping catheter, and may be configured to inject, via one or more electrodes, a current that is sunk at one or more electrodes of the magnetically tracked catheter. In either case, the one or more electrodes of the vascular mapping catheter may include independent electrodes coupled to a portion of the vascular mapping catheter, portions of the catheter itself (e.g., the conductive metal portion of a guidewire), and/or the like.

Each of the respective points at which a cardiac electrical signal is sensed may have a corresponding set of three-dimensional position coordinates. For example, the position coordinates of the points may be represented in Cartesian coordinates. Other coordinate systems can be used, as well. In embodiments, an arbitrary origin is used and the respective position coordinates are defined with respect to the arbitrary origin. In some embodiments, the points have non-uniform spacing, while in other embodiments, the points have uniform spacing. In embodiments, the point corresponding to each sensed cardiac electrical signal may be located on the epicardial surface of the heart, the endocardial surface of the heart and/or above and/or below the epicardial surface of the heart and/or the endocardial surface of the heart.

As shown in FIG. 4, embodiments of the method 400 include determining, from the electrical signals, a number of impedance measurements associated with one of more electrodes of a vascular mapping catheter (block 404) and accessing a field map (block 406). In embodiments, the field map may include expected impedance measurements determined based on previously-determined positions of the one or more electrodes of the magnetically tracked catheter. As shown in FIG. 4, the method further includes determining, based on the impedance measurements and the field map, a number of positions of the one or more electrodes of the vascular mapping catheter (block 408). An epicardial vascular structure may be generated based on the positions of the vascular mapping catheter (block 410). In embodiments, a processing unit may also be configured to determine one or more characteristics, based on at least one of the electrical signals and additional sensed signals, of the vessel modeled by the epicardial vascular structure. In embodiments, for example, impedance values may be used to determine one or more characteristics of the vessel. In embodiments, for example, diseased vessels may be at least partially restructured such as by calcification that results in narrowing of the vessel. Embodiments may facilitate identifying and/or classifying the calcification/narrowing of vessels using impedance, optical sensors, chemical sensors, metabolic sensors, and/or the like.

According to embodiments, for example, the method 400 may further include determining one or more diameters or other size dimensions of a vessel. This further information may facilitate providing more accurate representations of the vascular structure on a cardiac map. In embodiments, a diameter or size of a vessel may be measured and/or estimated using local impedance values from a multi-electrode catheter within the vessel. Relatively straightforward formulas may be applied to determine size dimensions such as, for example, *V*=*ρ*(*L²*/*R*), where *ρ* is the resistivity of blood, *L* is the distance between measuring electrodes, and *R* is the resistance measured between the measuring electrodes. In embodiments, cylindrical or tapered cone models may be used in the calculation estimation, and, in embodiments, a standard size template may be applied to the vessel.

Embodiments of the method 400 further include accessing an epicardial cardiac map (block 412) and annotating the epicardial cardiac map with a representation of the epicardial vascular structure to generate an epicardial vascular map (block 414). Embodiments of the method 400 further include facilitating presentation of the epicardial vascular map on a display device (block 416). In embodiments, an epicardial cardiac map may be generated (e.g., using a magnetically tracked catheter disposed within a space around the heart, within a chamber of the heart, etc., such as, for example, by inserting the catheter via a sub-xyphoid puncture) and/or annotated based, at least in part, on the impedance measurements.

In embodiments, the cardiac map may also be generated and/or annotated, at least in part, using any number of other signals, techniques, and/or the like. For example, embodiments may utilize impedance mapping techniques to generate and/or annotate one or more portions of the cardiac map such as, for example, an anatomical shell upon which electrical signal features are represented. In embodiments, a surface may be fitted on one or more of the points associated with the cardiac electrical signals to generate a shell representing the epicardial surface of one or more cardiac structures. In embodiments, a surface may also be fitted on one or more of the points associated with the electrical signals to generate a shell representing an endocardium surface or other excitable cardiac tissue.

Embodiments may include displaying annotations on the cardiac map that represent features, extracted from electrical signals and/or derived from other features, such as, for example, epicardial vascular structures, impedance measurements, activation times, minimum voltage values, maximum voltages values, maximum negative time-derivatives of voltages, instantaneous potentials, voltage amplitudes, dominant frequencies, peak-to-peak voltages, and/or the like. Epicardial vascular structures may be represented on the cardiac map and may be, or include, any features extracted from electrical signals and/or derived from one or more of such features. For example, a vascular structure, or characteristic thereof, may be represented by one or more colors, textures, and/or the like.

According to embodiments, a GUI used for presenting the map may include any number of different input tools for manipulating the map. For example, the GUI may include a play/pause button, a tool configured to facilitate manual selection of the histogram bin or bins, tools configured to facilitate manual adjustment of parameters (e.g., signal baseline definitions, thresholds, EGM characteristics, filters, etc.), and/or the like. In embodiments, for example, the GUI may include a selection tool that can facilitate refining selections of highlighted EGMs, select particular EGMs and/or activations, and/or the like.

Various modifications and additions can be made to the exemplary embodiments discussed without departing from the scope of the presently disclosed subject matter. For example, while the embodiments described above refer to particular features, the scope of this disclosure also includes embodiments having different combinations of features and embodiments that do not include all of the described features. Accordingly, the scope of the subject matter disclosed herein is intended to embrace all such alternatives, modifications, and variations as fall within the scope of the claims.

## Claims

1. A system for facilitating display of cardiac information associated with a heart of a patient, the system comprising:
a display device (170) configured to present an epicardial vascular map, the epicardial vascular map comprising an epicardial cardiac map (210, 306) annotated with a representation of an epicardial vascular structure (310); and
a processing unit (120, 200) configured to:
receive a plurality of electrical signals obtained from at least one of a vascular mapping catheter and a magnetically tracked catheter, wherein the vascular mapping catheter comprises one or more electrodes, and wherein the magnetically tracked catheter comprises one or more additional electrodes;
determine, from the electrical signals, a plurality of impedance measurements associated with the one or more electrodes of the vascular mapping catheter;
access a field map (208), the field map comprising expected impedance measurements determined based on determined positions of the one or more additional electrodes of the magnetically tracked catheter;
determine, based on the plurality of impedance measurements and the field map, a plurality of positions of the one or more electrodes of the vascular mapping catheter;
generate, based on the plurality of positions of the one or more electrodes of the vascular mapping catheter, the epicardial vascular structure (310);
access the epicardial cardiac map (210, 306);
annotate the epicardial cardiac map (210, 306) with the representation of the epicardial vascular structure (310) to generate the epicardial vascular
map; and facilitate display, via the display device, of the epicardial vascular map.

2. The system of claim 2, further comprising the vascular mapping catheter, wherein the vascular mapping catheter is configured to be inserted into a blood vessel associated with the heart.

3. The system of either of claims 1 or 2, further comprising:
the magnetically tracked catheter, wherein the magnetically tracked catheter is configured to be inserted into the patient's body; and
a tracking system configured to determine positions, within the patient's body, of the one or more additional electrodes of the magnetically tracked catheter.

4. The system of any of claims 1-3, wherein the vascular mapping catheter comprises a guidewire.

5. The system of claim 4, wherein the one or more electrodes of the guidewire comprise a portion of the guidewire.

6. The system of any of claims 1-5, wherein the tracking system is further configured to magnetically track positions of the vascular mapping catheter.

7. The system of any of claims 1-6, wherein the plurality of electrical signals are obtained by the one or more electrodes of the vascular mapping catheter, and wherein the plurality of electrical signals correspond to an energy field generated by at least one of the magnetically tracked catheter and a field generator.

8. The system of claim 7, wherein the field generator comprises one or more patches disposed external to the patient's body.

9. The system of any of claims 1-6, wherein the plurality of electrical signals are obtained by the one or more additional electrodes of the magnetically tracked catheter, and wherein the plurality of electrical signals correspond to an energy field generated by the vascular mapping catheter.

10. A computer-implemented method of facilitating display of epicardial vascular information associated with a heart of a subject, the method comprising:
receiving (402) a plurality of electrical signals obtained from at least one of a vascular mapping catheter and a magnetically tracked catheter, wherein the vascular mapping catheter comprises one or more electrodes, and wherein the magnetically tracked catheter comprises one or more additional electrodes;
determining (404) from the electrical signals, a plurality of impedance measurements associated with the one or more electrodes of the vascular mapping catheter;
accessing (406) a field map, the field map comprising expected impedance measurements determined based on determined positions of the one or more additional electrodes of the magnetically tracked catheter;
determining (408), based on the plurality of impedance measurements and the field map, a plurality of positions of the one or more electrodes of the vascular mapping catheter;
determining (410), based on the plurality of positions of the one or more electrodes of the vascular mapping catheter, the epicardial vascular structure;
accessing (412) the epicardial cardiac map;
annotating (414) the epicardial cardiac map with the representation of the epicardial vascular structure to generate the epicardial vascular map; and
facilitating (416) display, via a display device, of the epicardial vascular map.

11. The method of claim 10, wherein the vascular mapping catheter comprises a guidewire.

12. The method of claim 11, wherein the one or more electrodes of the guidewire comprise a portion of the guidewire.

13. The method of any of claims 10-12, wherein the plurality of electrical signals are obtained by the one or more electrodes of the vascular mapping catheter, and wherein the plurality of electrical signals correspond to an energy field generated by at least one of the magnetically tracked catheter and a field generator.

14. The method of any of claims 10-12, wherein the plurality of electrical signals are obtained by the one or more additional electrodes of the magnetically tracked catheter, and wherein the plurality of electrical signals correspond to an energy field generated by the vascular mapping catheter.

15. The method of any of claims 10-14, further comprising generating, using the magnetically tracked catheter, the epicardial cardiac map.

## Patentansprüche

1. System zum Unterstützen der Anzeige kardialer Informationen, assoziiert mit einem Herzen eines Patienten, wobei das System umfasst:
eine Anzeigevorrichtung (170), dazu ausgelegt, eine epikardiale vaskuläre Abbildung zu präsentieren, wobei die epikardiale vaskuläre Abbildung eine epikardiale Abbildung des Herzens (210, 306) umfasst, markiert mit einer Darstellung einer epikardialen vaskulären Struktur (310); und
eine Verarbeitungseinheit (120, 200), dazu ausgelegt:
eine Vielzahl von elektrischen Signalen zu empfangen, die von mindestens einem von einem vaskulären Abbildungskatheter und einem magnetisch verfolgten Katheter erhalten werden, wobei der vaskuläre Abbildungskatheter eine oder mehrere Elektroden umfasst, und wobei der magnetisch verfolgte Katheter eine oder mehrere zusätzliche Elektroden umfasst;
aus den elektrischen Signalen eine Vielzahl von Impedanzmesswerten zu bestimmen, die der einen oder mehreren Elektroden des vaskulären Abbildungskatheters zugeordnet sind;
auf eine Feldkarte (208) zuzugreifen, wobei die Feldkarte erwartete Impedanzmesswerte umfasst, die auf Grundlage bestimmter Positionen der einen oder mehrerer zusätzlicher Elektroden des magnetisch verfolgten Katheters bestimmt wurden;
auf Grundlage der Vielzahl von Impedanzmesswerten und der Feldkarte eine Vielzahl von Positionen der einen oder mehrerer Elektroden des vaskulären Abbildungskatheters zu bestimmen;
auf Grundlage der Vielzahl von Positionen der einen oder mehrerer Elektroden des vaskulären Abbildungskatheters die epikardiale vaskuläre Struktur (310) zu generieren;
auf die epikardiale Abbildung des Herzens (210, 306) zuzugreifen;
die epikardiale Abbildung des Herzens (210, 306) mit der Darstellung der epikardialen vaskulären Struktur (310) zu markieren, um die epikardiale vaskuläre Abbildung zu generieren; und
über die Anzeigevorrichtung die Anzeige der epikardialen vaskulären Abbildung zu ermöglichen.

2. System nach Anspruch 2, ferner umfassend den vaskulären Abbildungskatheter, wobei der vaskuläre Abbildungskatheter dazu ausgelegt ist, in ein dem Herzen zugeordnetes Blutgefäß eingesetzt zu werden.

3. System nach einem der Ansprüche 1 oder 2, ferner umfassend:
den magnetisch verfolgten Katheter, wobei der magnetisch verfolgte Katheter dazu ausgelegt ist, in den Körper des Patienten eingesetzt zu werden; und
ein Verfolgungssystem, das dazu ausgelegt ist, innerhalb des Körpers des Patienten Positionen der einen oder mehrerer zusätzlicher Elektroden des magnetisch verfolgten Katheters zu bestimmen.

4. System nach einem der Ansprüche 1-3, wobei der vaskuläre Abbildungskatheter einen Führungsdraht umfasst.

5. System nach Anspruch 4, wobei die eine oder mehrere Elektroden des Führungsdrahts einen Abschnitt des Führungsdrahts umfassen.

6. System nach einem der Ansprüche 1-5, wobei das Verfolgungssystem ferner dazu ausgelegt ist, Positionen des vaskulären Abbildungskatheters magnetisch zu verfolgen.

7. System nach einem der Ansprüche 1-6, wobei die Vielzahl von elektrischen Signalen durch die eine oder mehrere Elektroden des vaskulären Abbildungskatheters erhalten werden, und wobei die Vielzahl von elektrischen Signalen einem Energiefeld entsprechen, das durch mindestens einen von dem magnetisch verfolgten Katheter und einem Feldgenerator erzeugt wird.

8. System nach Anspruch 7, wobei der Feldgenerator ein oder mehrere Patches umfasst, die außerhalb des Körpers des Patienten angeordnet sind.

9. System nach einem der Ansprüche 1-6, wobei die Vielzahl von elektrischen Signalen durch die eine oder mehrere zusätzliche Elektroden des magnetisch verfolgten Katheters erhalten werden, und wobei die Vielzahl von elektrischen Signalen einem durch den vaskulären Abbildungskatheter erzeugten Energiefeld entsprechen.

10. Computerimplementiertes Verfahren zum Ermöglichen der Anzeige von epikardialen vaskulären Informationen, die einem Herzen eines Subjekts zugeordnet sind, wobei das Verfahren umfasst:
Empfangen (402) einer Vielzahl von elektrischen Signalen, die von mindestens einem von einem vaskulären Abbildungskatheter und einem magnetisch verfolgten Katheter erhalten werden, wobei der vaskuläre Abbildungskatheter eine oder mehrere Elektroden umfasst, und wobei der magnetisch verfolgte Katheter eine oder mehrere zusätzliche Elektroden umfasst;
Bestimmen (404), aus den elektrischen Signalen, einer Vielzahl von Impedanzmesswerten, die der einen oder mehreren Elektroden des vaskulären Abbildungskatheters zugeordnet sind;
Zugreifen (406) auf eine Feldkarte, wobei die Feldkarte erwartete Impedanzmesswerte umfasst, die auf Grundlage bestimmter Positionen der einen oder mehrerer zusätzlicher Elektroden des magnetisch verfolgten Katheters bestimmt wurden;
Bestimmen (408), auf Grundlage der Vielzahl von Impedanzmesswerten und der Feldkarte, einer Vielzahl von Positionen der einen oder mehrerer Elektroden des vaskulären Abbildungskatheters;
Bestimmen (410), auf Grundlage der Vielzahl von Positionen der einen oder mehrerer Elektroden des vaskulären Abbildungskatheters, der epikardialen vaskulären Struktur;
Zugreifen (412) auf die epikardiale Abbildung des Herzens;
Markieren (414) der epikardialen Abbildung des Herzens mit der Darstellung der epikardialen vaskulären Struktur, um die epikardiale vaskuläre Abbildung zu generieren; und
Ermöglichen (416), über die Anzeigevorrichtung, der Anzeige der epikardialen vaskulären Abbildung.

11. Verfahren nach Anspruch 10, wobei der vaskuläre Abbildungskatheter einen Führungsdraht umfasst.

12. Verfahren nach Anspruch 11, wobei die eine oder mehrere Elektroden des Führungsdrahts einen Abschnitt des Führungsdrahts umfassen.

13. Verfahren nach einem der Ansprüche 10-12, wobei die Vielzahl von elektrischen Signalen durch die eine oder mehrere Elektroden des vaskulären Abbildungskatheters erhalten werden, und wobei die Vielzahl von elektrischen Signalen einem Energiefeld entsprechen, das durch mindestens einen von dem magnetisch verfolgten Katheter und einem Feldgenerator erzeugt wird.

14. Verfahren nach einem der Ansprüche 10-12, wobei die Vielzahl von elektrischen Signalen durch die eine oder mehrere zusätzliche Elektroden des magnetisch verfolgten Katheters erhalten werden, und wobei die Vielzahl von elektrischen Signalen einem durch den vaskulären Abbildungskatheter erzeugten Energiefeld entsprechen.

15. Verfahren nach einem der Ansprüche 10-14, ferner umfassend das Erzeugen der epikardialen Abbildung des Herzens unter Verwendung des magnetisch verfolgten Katheters.

## Revendications

1. Système pour faciliter l'affichage d'une information cardiaque qui est associée au cœur d'un patient, le système comprenant :
un dispositif d'affichage (170) qui est configuré pour présenter une carte vasculaire épicardique, la carte vasculaire épicardique comprenant une carte cardiaque épicardique (210, 306) qui est annotée avec une représentation d'une structure vasculaire épicardique (310) ; et
une unité de traitement (120, 200) qui est configurée pour :
recevoir une pluralité de signaux électriques qui sont obtenus à partir d'au moins un cathéter pris parmi un cathéter de cartographie vasculaire et un cathéter suivi magnétiquement, dans lequel le cathéter de cartographie vasculaire comprend une ou plusieurs électrode(s) et dans lequel le cathéter suivi magnétiquement comprend une ou plusieurs électrode(s) additionnelle(s) ;
déterminer, à partir des signaux électriques, une pluralité de mesures d'impédance qui sont associées aux une ou plusieurs électrodes du cathéter de cartographie vasculaire ;
accéder à une carte de champ (208), la carte de champ comprenant des mesures d'impédance attendues qui sont déterminées sur la base de positions déterminées des une ou plusieurs électrodes additionnelles du cathéter suivi magnétiquement ;
déterminer, sur la base de la pluralité de mesures d'impédance et de la carte de champ, une pluralité de positions des une ou plusieurs électrodes du cathéter de cartographie vasculaire ;
générer, sur la base de la pluralité de positions des une ou plusieurs électrodes du cathéter de cartographie vasculaire, la structure vasculaire épicardique (310) ;
accéder à la carte cardiaque épicardique (210, 306) ;
annoter la carte cardiaque épicardique (210, 306) avec la représentation de la structure vasculaire épicardique (310) pour générer la carte vasculaire épicardique ; et
faciliter l'affichage, via le dispositif d'affichage, de la carte vasculaire épicardique.

2. Système selon la revendication 2, comprenant en outre le cathéter de cartographie vasculaire, dans lequel le cathéter de cartographie vasculaire est configuré pour être inséré à l'intérieur d'un vaisseau sanguin qui est associé au cœur.

3. Système selon l'une quelconque des revendications 1 ou 2, comprenant en outre :
le cathéter suivi magnétiquement, dans lequel le cathéter suivi magnétiquement est configuré pour être inséré à l'intérieur du corps du patient ; et
un système de suivi qui est configuré pour déterminer des positions, à l'intérieur du corps du patient, des une ou plusieurs électrodes additionnelles du cathéter suivi magnétiquement.

4. Système selon l'une quelconque des revendications 1 à 3, dans lequel le cathéter de cartographie vasculaire comprend un fil de guidage.

5. Système selon la revendication 4, dans lequel les une ou plusieurs électrodes du fil de guidage comprennent une partie du fil de guidage.

6. Système selon l'une quelconque des revendications 1 à 5, dans lequel le système de suivi est en outre configuré pour suivre magnétiquement des positions du cathéter de cartographie vasculaire.

7. Système selon l'une quelconque des revendications 1 à 6, dans lequel les signaux électriques de la pluralité de signaux électriques sont obtenus au moyen des une ou plusieurs électrodes du cathéter de cartographie vasculaire et dans lequel les signaux électriques de la pluralité de signaux électriques correspondent à un champ d'énergie qui est généré par au moins un composant pris parmi le cathéter suivi magnétiquement et un générateur de champ.

8. Système selon la revendication 7, dans lequel le générateur de champ comprend un ou plusieurs patch(s) qui est/sont disposé(s) de façon externe par rapport au corps du patient.

9. Système selon l'une quelconque des revendications 1 à 6, dans lequel les signaux électriques de la pluralité de signaux électriques sont obtenus au moyen des une ou plusieurs électrodes additionnelles du cathéter suivi magnétiquement et dans lequel les signaux électriques de la pluralité de signaux électriques correspondent à un champ d'énergie qui est généré par le cathéter de cartographie vasculaire.

10. Procédé mis en œuvre par ordinateur de facilitation de l'affichage d'une information vasculaire épicardique qui est associée au cœur d'un sujet, le procédé comprenant :
la réception (402) d'une pluralité de signaux électriques qui sont obtenus à partir d'au moins un cathéter pris parmi un cathéter de cartographie vasculaire et un cathéter suivi magnétiquement, dans lequel le cathéter de cartographie vasculaire comprend une ou plusieurs électrode(s) et dans lequel le cathéter suivi magnétiquement comprend une ou plusieurs électrode(s) additionnelle(s) ;
la détermination (404), à partir des signaux électriques, d'une pluralité de mesures d'impédance qui sont associées aux une ou plusieurs électrodes du cathéter de cartographie vasculaire ;
l'accès (406) à une carte de champ, la carte de champ comprenant des mesures d'impédance attendues qui sont déterminées sur la base de positions déterminées des une ou plusieurs électrodes additionnelles du cathéter suivi magnétiquement ;
la détermination (408), sur la base de la pluralité de mesures d'impédance et de la carte de champ, d'une pluralité de positions des une ou plusieurs électrodes du cathéter de cartographie vasculaire ;
la détermination (410), sur la base de la pluralité de positions des une ou plusieurs électrodes du cathéter de cartographie vasculaire, de la structure vasculaire épicardique ;
l'accès (412) à la carte cardiaque épicardique ;
l'annotation (414) de la carte cardiaque épicardique avec la représentation de la structure vasculaire épicardique pour générer la carte vasculaire épicardique ; et
la facilitation (416) de l'affichage, via un dispositif d'affichage, de la carte vasculaire épicardique.

11. Procédé selon la revendication 10, dans lequel le cathéter de cartographie vasculaire comprend un fil de guidage.

12. Procédé selon la revendication 11, dans lequel les une ou plusieurs électrodes du fil de guidage comprennent une partie du fil de guidage.

13. Procédé selon l'une quelconque des revendications 10 à 12, dans lequel les signaux électriques de la pluralité de signaux électriques sont obtenus au moyen des une ou plusieurs électrodes du cathéter de cartographie vasculaire et dans lequel les signaux électriques de la pluralité de signaux électriques correspondent à un champ d'énergie qui est généré par au moins un composant pris parmi le cathéter suivi magnétiquement et un générateur de champ.

14. Procédé selon l'une quelconque des revendications 10 à 12, dans lequel les signaux électriques de la pluralité de signaux électriques sont obtenus au moyen des une ou plusieurs électrodes additionnelles du cathéter suivi magnétiquement et dans lequel les signaux électriques de la pluralité de signaux électriques correspondent à un champ d'énergie qui est généré par le cathéter de cartographie vasculaire.

15. Procédé selon l'une quelconque des revendications 10 à 14, comprenant en outre la génération, en utilisant le cathéter suivi magnétiquement, de la carte cardiaque épicardique.
